(19) Europäisches Patentamt
European Patent Office
Office européen des brevets

(11) Veröffentlichungsnummer: **0 009 150**
**B1**

# (12) EUROPÄISCHE PATENTSCHRIFT

(45) Veröffentlichungstag der Patentschrift: **02.09.81**

(51) Int. Cl.³: **C 07 C 45/50,** C 07 C 47/02

(21) Anmeldenummer: **79103218.8**

(22) Anmeldetag: **31.08.79**

(54) **Verfahren zur Herstellung von Aldehyden.**

(30) Priorität: **15.09.78 DE 2840168**

(43) Veröffentlichungstag der Anmeldung: **02.04.80 Patentblatt 80/7**

(45) Bekanntmachung des Hinweises auf die Patenterteilung: **02.09.81 Patentblatt 81/35**

(84) Benannte Vertragsstaaten: **BE CH DE FR GB IT NL SE**

(56) Entgegenhaltungen:
**FR-A-2 314 910**
**US-A-3 816 337**

(73) Patentinhaber: **BASF Aktiengesellschaft, Carl-Bosch-Strasse 38, D-6700 Ludwigshafen (DE)**

(72) Erfinder: **Richter, Wolfgang, Dr. Dipl.-Chem., Keplerstrasse 38, D-6700 Ludwigshafen (DE)**
Erfinder: **Kummer, Rudolf, Dr. Dipl.-Chem., Kreuzstrasse 6, D-6710 Frankenthal (DE)**
Erfinder: **Schwirten, Kurt, Dr. Dipl.-Chem., Prager Strasse 27, D-6700 Ludwigshafen (DE)**

**EP 0 009 150 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Verfahren zur Herstellung von Aldehyden

Die vorliegende Erfindung betrifft ein verbessertes Verfahren zur Herstellung von Aldehyden durch Hydroformylierung der entsprechenden olefinisch ungesättigten Verbindungen mit Hilfe von Rhodium-Carbonyl-Komplexverbindungen, welche tertiäre Phosphine als Liganden enthalten.

Die Hydroformylierung olefinisch ungesättigter Verbindungen mit Hilfe von Cobalt- oder Rhodium-Carbonylkomplexen als CO-Transferkatalysatoren ist allgemein bekannt.

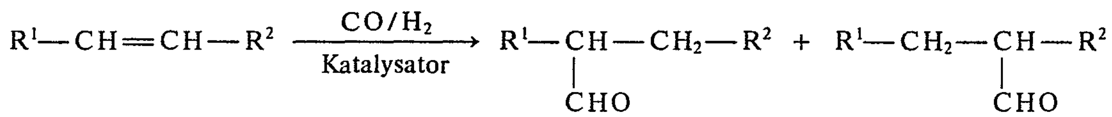


$R^1$, $R^2$: H; org. Reste

Weiterhin ist allgemein bekannt, daß hierbei die Verwendung von Rhodium-Carbonyl-Komplexen, welche tertiäre Phospine als Liganden enthalten, im Hinblick auf die Verfahrensergebnisse und in verfahrenstechnischer Hinsicht besondere Vorteile bietet. Einer dieser Vorteile besteht darin, daß die genannten Katalysatoren auch bei Normaldruck sehr thermostabil sind, so daß man die Verfahrensprodukte aus dem Reaktionsgemisch abdestillieren kann, ohne daß sich diese Katalysatoren zersetzen. Sie verbleiben im hochsiedenden Rückstand, der in die Hydroformylierungsstufe zurückgeführt werden kann.

Da die Menge des Rückstandes auf diese Weise naturgemäß ständig zunimmt, ist von Zeit zu Zeit dessen Aufbereitung erforderlich, etwa indem man ihn mit wäßrigen Peroxiden und Salpetersäure erhitzt und die hierbei größtenteils in die wäßrige Phase gelangenden Rhodiumsalze nach an sich bekannten Methoden wieder in die aktiven Komplexe oder deren Vorstufen überführt. Es liegt auf der Hand, daß es sich hierbei und bei ähnlichen Aufbereitungsverfahren, z. B. durch Extraktion oder Verbrennung, um lästige und aufwendige Nebenoperationen handelt, die man an sich nach Möglichkeit zu vermeiden oder zumindest einzuschränken bemüht ist. Um so nachteiliger ist es, daß die Rückstandsaufbereitung aber nicht nur dann erforderlich ist, wenn die Menge des Rückstandes nicht mehr tolerierbar ist, sondern bereits deswegen, weil die Aktivität des Katalysators im Laufe von etwa 1000 Betriebsstunden auf einen Wert absinkt, von dem an die Hydroformylierung wegen der dann erforderlichen zu langen Reaktionszeiten unwirtschaftlich wird.

Die Ursachen des Aktivitätsverlustes sind im einzelnen noch weitgehend ungeklärt, jedoch ist anzunehmen, daß es sich stets um eine Verdrängung der beweglichen Carbonylgruppen durch stabilere Liganden handelt, so daß kein CO-Transfer mehr möglich ist. Diese Annahme steht z. B. mit der Tatsache in Einklang, daß die als Ligand besonders stabile Cyanidgruppe ein entsprechend starkes Katalysatorgift ist. In abgeschwächter Form gilt dies auch für Halogen- und Sulfidionen.

Aus der US-PS 3 816 337 sind Katalysatorsysteme für die Hydroformylierung bekannt, die als aktive Metalle Cobalt einerseits und ein Metall der I. oder II. Gruppe des Periodensystems, darunter Zink, andererseits enthalten. Diese Katalysatorsysteme bewirken nicht nur die Hydroformylierung des Olefins, sondern darüber hinaus die Dimerisierung (Aldolkondensation) des entstehenden Aldehyds sowie eine anschließende Dehydratisierung des Dimerisierungsproduktes.

Ferner ist aus der FR-PS 2 314 910 die Hydroformylierung von Olefinen mittels wäßriger Lösungen von wasserlöslichen Rhodiumkomplexen bekannt. Die Wasserlöslichkeit dieser Komplexe wird durch Triarylphosphine als Liganden bewirkt, welche $SO_3^{\ominus}$-Gruppen am Arylrest tragen, wobei u. a. Zink und Kupfer als Kationen dienen können. Diese spezielle Verfahrensweise schließt jedoch die Verwendung von wasserunlöslichen Phosphinen aus.

Aufgabe der Erfindung war es, die Aktivität des Katalysators so weit zu erhalten, daß aus Gründen nachlassender Aktivität keine Aufbereitung der hochsiedenden katalysatorhaltigen Rückstände mehr erforderlich ist.

Es wurde nun ein verbessertes Verfahren zur Herstellung von Aldehyden durch Hydroformylierung der entsprechenden olefinisch ungesättigten Verbindungen mit Hilfe von Rhodium-Carbonyl-Komplexverbindungen, die tertiäre Phosphine der allgemeinen Formel $PR_3$ als Liganden enthalten, wobei die Reste R, die gleich oder verschieden sein können, Kohlenwasserstoffreste mit 4–12 C-Atomen bedeuten, gefunden, welches dadurch gekennzeichnet ist, daß man diese Reaktion in Gegenwart von solchen, im Reaktionsgemisch löslichen Verbindungen des Kupfers, Silbers oder Zinks vornimmt, die eine Aktivierung des desaktivierten Rhodium-Carbonyl-Komplexes bewirken.

Es wurde weiterhin gefunden, daß sich Verbindungen des Kupfers und des Zinks für den genannten Zweck besonders gut eignen. Als definitionsgemäße Verbindungen der genannten Metalle können

Salze, Oxide und Komplexverbindungen mit Ausnahme solcher Verbindungen verwendet werden, deren Komponenten nicht ihrerseits desaktivierend auf den Rhodiumkatalysator wirken. Demgemäß sind die Halogenide, Sulfide und Cyanide beispielsweise, wie sich allerdings von selbst versteht, ungeeignet. Brauchbar hingegen sind u. a. die Sulfate, Phosphate und vor allem die fettsauren Salze wie die Acetate, Propionate, Butyrate oder Stearate. Auch die Oxide oder Hydroxide können eingesetzt werden, da diese mit den stets im Hydroformylierungsgemisch vorliegenden organischen Säuren unter den Reaktionsbedingungen in die Salze dieser Säuren übergehen. Ferner kommen die ebenfalls gut geeigneten Komplexverbindungen wie die Acetylacetonate und Acetoacetate in Betracht.

Ob eine Verbindung geeignet ist, läßt sich durch einfache Modellversuche unschwer ermitteln, indem man eine Test-Hydroformylierung in Gegenwart eines mit Sulfid oder Chlorid gänzlich desaktivierten Rhodium-Katalysators vornimmt; die Reaktionsgeschwindigkeit ist in diesem Fall praktisch Null, d. h., es findet keine Umsetzung von Olefin zum Aldehyd statt. Eine Verbindung eines der genannten Metalle ist dann geeignet, wenn die Hydroformylierung nach ihrer Zugabe, eventuell nach einer kurzen Induktionsperiode, die Reaktionsgeschwindigkeit erreicht, die mit einem frischen, nicht desaktivierten Rh-Katalysator erzielt wird. Die Reaktionsgeschwindigkeit läßt sich hierbei leicht über den Verbrauch des $CO/H_2$-Gasgemisches ermitteln. Es sei indes betont, daß bis auf wenige Verbindungsklassen (z. B. Halogenide, Cyanide und Sulfide) die meisten Verbindungen der genannten Metalle den erfindungsgemäßen Zweck erfüllen. Vermutlich wirken die Metallverbindungen in der Weise, daß die betreffenden Metalle eine stärkere Affinität zu den inhibierenden Substanzen, z. B. Halogeniden, Sulfiden und Cyaniden, haben als das Rhodium. Dementsprechend richtet sich die Menge der wirksamen Metallverbindungen — sie bezieht sich zweckmäßigerweise stets auf das Metall — nach der Menge der inhibierenden Substanzen. Da es sich meist um sehr geringe Mengen handelt und da auch die Art der Inhibitoren nicht genau bekannt ist, muß diese wirksame Menge von Fall zu Fall empirisch bestimmt werden. Ein Überschuß der Metallverbindung schadet allerdings nach den bisherigen Beobachtungen nicht.

Als Regel für Hydroformylierungen von Olefinen üblicher technischer Reinheit gilt ein Atomverhältnis von Rhodium zu einem der genannten Metalle von 1 : 0,1 bis 1 : 20, woraus sich die Menge der Metallverbindung errechnet. Diese Menge reicht aus, die unverminderte Aktivität des Rhodiumkatalysators für ungefähr 4000 Betriebsstunden zu gewährleisten, also etwa für die Zeit, nach welcher eine Katalysatorregenerierung aus Gründen der Entfernung des hochsiedenden Rückstandes, der sich bis dahin im Verfahrenskreislauf akkumuliert hat, ohnehin erforderlich ist.

Nimmt man die Katalysatorregenerierung in der Weise vor, daß man den Rückstand mit einem wäßrigen Gemisch aus Salpetersäure und Wasserstoffperoxid auskocht, so gelangt nicht nur das Rhodium als Rhodiumnitrat in die wäßrige Phase, sondern auch das mitverwendete Metall als dessen Nitrat.

Mit einem tertiären Phosphin und in Gegenwart von $H_2$ und CO bei 1—5 bar läßt sich hierbei der Rhodiumkomplex ausfällen, der dann in die Hydroformylierungsstufe zurückgeführt wird. Sofern es nicht wirtschaftlicher ist, die verbleibenden Metallnitratlösungen zu verwerfen, kann man die Nitrate durch Eindampfen isolieren, diese gegebenenfalls durch Erhitzen in die Oxide überführen, die Oxide in einer organischen Säure auflösen und diese Lösungen ebenfalls wieder in den Kreislauf zurückgeben.

Der Erfolg des erfindungsgemäßen Verfahrens ist von der Art der Hydroformylierung, also von der Art des eingesetzten Olefins, unabhängig, so daß sich eine Aufzählung der hierbei ausgeübten oder möglichen Verfahren erübrigt. Besondere Bedeutung hat das Verfahren indes naturgemäß bei großtechnischen Synthesen von Industriechemikalien wie bei der Hydroformylierung von Propylen zu n-Butyraldehyd (und daneben iso-Butyraldehyd) sowie der Hydroformylierung von Äthylen zu Propionaldehyd, da es hier auf äußerste Wirtschaftlichkeit ankommt. Allgemein nimmt man die Hydroformylierung mittels der Rhodiumkatalysatoren bei einem Druck von 1—30 bar und Temperaturen zwischen 70 und 150°C mit einem äquimolaren oder annähernd äquimolaren Gemisch aus CO und $H_2$ vor.

Die bevorzugten Rhodium-Katalysatoren haben die allgemeine Formel

$$HRh(CO)(PR_3)_3$$

wobei die Reste R, die gleich oder verschieden sein können, für Kohlenwasserstoffreste mit 4—12 C-Atomen stehen. Technisch besondere Bedeutung hat die Phenylgruppe (Ph) als Rest R, darunter vor allem das Triphenylphosphin ($PPh_3$) als Ligand. Die Art der tertiären Phosphine hat im übrigen jedoch keinen erkennbaren Einfluß auf die erfindungsgemäße Verfahrensverbesserung. Man kann das Rhodium in Form der genannten Komplexe einsetzen oder auch in Form eines Rhodiumsalzes wie Rhodiumnitrat und den entsprechenden Mengen des Phosphins. Im letzteren Falle bilden sich die aktiven Komplexe unter den Hydroformulierungsbedingungen in situ. Ferner kann es sich empfehlen, das Phosphin in einem bis zu 100fachen stöchiometrischen Überfluß einzusetzen.

Die Konzentration des Katalysators, berechnet auf den Metallgehalt, beträgt, wie bei der Rh-katalysierten Hydroformulierung allgemein üblich, zwischen 10 und 1000 ppm des Reaktionsgemisches.

Beispiel 1

In einem Autoklaven von 2 l Inhalt wurden jeweils 80 g Oct-1-en, gelöst in 250 ml Toluol, in Gegenwart von 2 g Triphenylphosphin und 5 mg Rhodium in Form des Komplexes $HRh(CO)(PPh_3)_3$ bei 100°C und unter einem Druck von 10 bar eines äquimolaren $CO/H_2$-Gemisches hydroformyliert, und zwar

a) mit einem frisch bereiteten Katalysator
b) mit einem Katalysator, der in 8 g eines hochsiedenden Rückstandes enthalten war, der aus der Hydroformylierung von Äthylen stammte
c) mit der Katalysatorlösung (b), die zusätzlich 100 mg Cu-(II)-acetylacetonat enthielt (Rh : Cu = 1 : 8) und
d) mit der Katalysatorlösung (b), die zusätzlich Zinkacetylacetonat enthielt (Rh : Zn = 1 : 8)

Im Falle (a) wurde nach 1,5 Stunden kein Gas mehr aufgenommen, d. h., nach dieser Zeit war ein praktisch 100%iger Umsatz erreicht. In dieser Zeit wurde mit dem Katalysator (b) lediglich ein Umsatz von etwa 50% erzielt. Im Falle des Katalysatorsystems mit Kupfer (Fall c) war der Umsatz ebenfalls praktisch vollständig, wogegen dieser optimale Wert mit Zink (Fall d) mit etwa 95% des Umsatzes in dieser Zeit nicht ganz erreicht wurde.

Beispiel 2

In einem Modellversuch unter den gleichen Bedingungen wie in Beispiel 1 wurde statt des dort genannten Rh-Komplexes der Komplex $ClRh(CO)(PPh_3)_2$ verwendet, und zwar

a) für sich allein
b) in Gegenwart von Kupfernonanat und
c) in Gegenwart von Zinknonanat

Nach 1,5 Stunden wurde im Falle (a) nur ein Umsatz von 10% erzielt, während er in den Fällen (b) und (c), wie in Beispiel 1, praktisch 100% betrug.

Das gleiche Bild bot sich bei Verwendung des Katalysators $HRh(CO)(PPh_3)_3$, der vor seinem Einsatz der Einwirkung von Chlorwasserstoff ausgesetzt wurde.

Beispiel 3

In einem Modellversuch unter den gleichen Bedingungen und mit den gleichen molaren Mengenverhältnissen wie in Beispiel 1, wurde der Katalysator $HRh(CO)(PPh_3)_3$ verwendet, der vor seinem Einsatz mit Schwefelwasserstoff begast wurde. Dieser desaktivierte Katalysator wurde

a) für sich allein und
b) zusammen mit Kupfer-II-acetylacetonat

verwendet.

Im Falle (a) fand überhaupt kein Umsatz statt, während die Reaktion im Falle (b) nach kurzer Induktionsperiode einsetzte und nach 1,7 Stunden den Umsatz von rund 100% erreichte.

**Patentanspruch**

Verfahren zur Herstellung von Aldehyden durch Hydroformylierung der entsprechenden olefinisch ungesättigten Verbindungen mit Hilfe von Rhodium-Carbonyl-Komplexverbindungen, die tertiäre Phosphine der allgemeinen Formel $PR_3$ als Liganden enthalten, wobei die Reste R, die gleich oder verschieden sein können, Kohlenwasserstoffreste mit 4—12 C-Atomen bedeuten, dadurch gekennzeichnet, daß man diese Reaktion in Gegenwart von solchen im Reaktionsgemisch löslichen Verbindungen des Kupfers, Silbers oder Zinks vornimmt, die eine Aktivierung des desaktivierten Rhodium-Carbonyl-Komplexes bewirken.

**Claim**

A process for the preparation of aldehydes by hydroformylating the corresponding olefinically unsaturated compounds with the aid of rhodium-carbonyi complexes which contain tertiary

phosphines of the general formula $PR_3$ as ligands, the radicals R, which may be identical or different, being hydrocarbon radicals of 4 to 12 carbon atoms, characterized in that this reaction is carried out in the presence of compounds of copper, silver or zinc which are soluble in the reaction mixture and activate the deactivated rhodium-carbonyl complexes.

## Revendication

Procédé de préparation d'aldéhydes par hydroformylation des composés à insaturation oléfinique correspondants à l'aide de composés complexes de rhodium-carbonyle contenant des phosphines tertiaires de formule générale $PR_3$ en tant que ligands, les symboles R, ayant des significations identiques ou différentes, représentant des restes hydrocarbonés en C4–C12, caractérisé en ce que l'on effectue cette réaction en présence de composés du cuivre, de l'argent ou du zinc, solubles dans le mélange de réaction, qui provoquent une activation du complexe de rhodium-carbonyle désactivé.